(19)

Europäisches
Patentamt
European
Patent Office
Office européen
des brevets

(11) **EP 3 428 639 A1**

(12) **EUROPEAN PATENT APPLICATION**

(43) Date of publication:
**16.01.2019 Bulletin 2019/03**

(51) Int Cl.:
**G01N 33/00** (2006.01)   **G01N 15/14** (2006.01)
**G01N 21/00** (2006.01)   **G01N 15/00** (2006.01)
**B01D 46/44** (2006.01)

(21) Application number: **17181221.7**

(22) Date of filing: **13.07.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **KELLY, Declan Patrick
  5656 AE Eindhoven (NL)**

• **RONDA, Cornelis Reinder
  5656 AE Eindhoven (NL)**
• **CHEN, Weizhong
  5656 AE Eindhoven (NL)**
• **BOUMA, Peter Hermanus
  5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **AIR POLLUTANT SENSING**

(57)   A device (10, 100) is disclosed for determining an ambient air pollutant concentration. The device comprises a processor (15) adapted to receive a sensor signal from an air pollutant sensor (120) of a portable air pollutant sensor device (100) comprising an air flow channel (110) extending between a first opening (111) open to an ambient environment including the ambient air pollutant concentration and a second opening (113) open to said ambient environment, the air flow channel comprising a sensing area (115) including the air pollutant sensor (120) and a heating element (140) for inducing an air flow through said air flow channel; receive a further sensor signal from an orientation sensor (130) arranged to determine an orientation of the portable air pollutant sensor device; determine an orientation of the portable air pollutant sensor device from the received further sensor signal; compare the determined orientation of the portable air pollutant sensor with a defined range of orientations (220) including an optimal orientation (210) of the portable air pollutant sensor device for sensing said ambient air pollutant concentration; determine the ambient air pollutant concentration only if the determined orientation of the portable air pollutant sensor lies within the defined range of orientations by determining an actual air pollutant count from the received sensor signal and correcting the actual air pollutant count based on the determined orientation of the portable air pollutant sensor if the determined orientation differs from the optimal orientation. A computer program product for configuring such a device is also disclosed.

FIG. 1

**Description**

FIELD OF THE INVENTION

The present invention relates to a device for determining an ambient air pollutant concentration, the device comprising a processor adapted to receive a sensor signal from an air pollutant sensor of a portable air pollutant sensor device comprising an air flow channel extending between a first opening open to an ambient environment including the ambient air pollutant concentration and a second opening open to said ambient environment, the air flow channel comprising a sensing area including the air pollutant sensor and a heating element for inducing an air flow through said air flow channel.

The present invention further relates to a computer program product for such a device.

BACKGROUND OF THE INVENTION

With increasing concerns over the adverse health effects of air pollution e.g. PM2.5 (particles smaller than 2.5 $\mu$m diameter range) on human health, the demand for environmental pollution information, e.g. pollution monitoring of living spaces is continuously increasing. For example, in some developing countries such as China and India, severe pollution such as PM2.5 pollution has become a common problem over the last decade. This has caused people to become increasingly interested in obtaining information concerning environmental pollution levels, which interest has led to an increased demand for portable particle sensor devices, e.g. air pollutant sensor devices integrated in consumer electronic devices such as smart phones or other smart devices or wearable air pollutant sensors that may autonomously determine the ambient levels of an air pollutant of interest, e.g. particulate matter such as PM2.5 or may communicate with a computing device such as a smart phone or tablet to have the sensor signals processed by the computing device.

In order to approximate the ambient air pollutant concentration, the volume of air passing through the air pollutant sensor during a sensing event has to be approximated. This requires control of the air flow speed through the air pollutant sensor. Although it is possible to measure the air flow speed through an air flow channel in which such sensors are located, e.g. with an air flow meter, this is often not preferred due to the need to integrate an additional component in the air pollutant sensor, which adds to the cost of the sensor device and increases its form factor, which is undesirable when the sensor device needs to be portable.

A commonly used approach to generate a controlled air flow through the air flow channel of such sensor devices is to use a resistive heating element in the air flow channel. The heat generated by such a resistive heating element induces a temperature gradient between the air in the air flow channel heated by this heat and ambient air, which induces an air flow through the air flow channel. As the amount of heat that is generated in this manner can be controlled by the amount of current that is passed through the resistive heating element, the magnitude of the temperature gradient and the resulting air flow speed can be accurately approximated.

JP 2016/212024 A discloses a particle detection device integrated in an air purifier through which an air flow is induced with a resistive heating element. For this particle detection device it was found that a degree of inclination of the air purifier under which the air purifier was installed affected the accuracy of the particle concentration determination with the particle detection device due to this inclination affecting the air flow speed through the particle detection device induced with the resistive heating element. In an embodiment, the air purifier includes an inclination detection means, which detects the angle of inclination of the air purifier, which detected angle of inclination is used to apply a correction factor to the particle count determined with the particle detection device in order to compensate for the influence of this inclination angle on the air flow rate through the particle detection device.

In JP 2016/212024 A, the angle of inclination of the air purifier typically is only determined once, after installation of the air purifier after which the correction factor is applied based on the assumption that the orientation of the air purifier post-installation will not change. However, this approach cannot be deployed to approximate the ambient concentration with an air pollutant sensor in a portable sensing device owing to the more dynamic nature of the orientation of such a portable sensing device.

SUMMARY OF THE INVENTION

The present invention seeks to provide a device for determining an ambient air pollutant concentration comprising a processor configured to accurately determine the ambient air pollutant concentration with such a portable air pollutant sensor device.

The present invention further seeks to provide a computer program product that can configure the processor of this device to accurately determine the ambient air pollutant concentration with such a portable air pollutant sensor device.

According to an aspect, there is provided a device for determining an ambient air pollutant concentration, the device comprising a processor adapted to receive a sensor signal from an air pollutant sensor of a portable air pollutant sensor device comprising an air flow channel extending between a first opening open to an ambient environment including the ambient air pollutant concentration and a second opening open to said ambient environment, the air flow channel comprising a sensing area including the air pollutant sensor and a heating element for inducing an air flow through said air flow channel; receive a further sensor signal from an orientation sensor arranged to determine an orientation of the portable air pollutant sensor device; determine an orientation of the portable air pollutant sensor device from the received further sensor signal; compare the determined orientation of the portable air pollutant sensor with a defined range of orientations including an optimal orientation of the portable air pollutant sensor device for sensing said ambient air pollutant concentration; determine the ambient air pollutant concentration only if the determined orientation of the portable air pollutant sensor lies within the defined range of orientations by determining an actual air pollutant count from the received sensor signal and correcting the actual air pollutant count based on the determined orientation of the portable air pollutant sensor if the determined orientation differs from the optimal orientation.

The present invention is based on the insight that an air pollutant sensor through which an air flow is generated with a heating element, e.g. a resistive heating element, typically can only operate accurately within a certain range of orientation angles of the air pollutant sensor device, e.g. its air flow channel. In other words, it has been determined that the rate of deviation of the actual air flow speed through the air flow channel from the theoretical air flow speed increases with increasing deviation of the actual orientation of the air pollutant sensor device from its desired (optimal) orientation such as a vertical orientation, such that at large deviations from this optimal orientation the air flow speed through the air flow channel of the air pollutant sensor device becomes highly sensitive to small variations in the orientation of the air pollutant sensor device, which makes it practically impossible to accurately estimate the actual air flow speed through the air flow channel of the air pollutant sensor device. In other words, although a degree of approximation is typically present in the determination of the air flow speed through such an air flow channel, e.g. due to fluctuations in the ambient temperature, the uncertainty in the approximation increases with increasing deviations from this optimal orientation such that at large deviations from this optimal orientation the determined air pollutant concentration becomes unreliable.

Hence, embodiments of the present invention ensured that only reliable air pollutant sensor readings are obtained by only considering those sensor readings obtained with the air pollutant sensor device in an orientation within an orientation range around the optical orientation of the air pollutant sensor device for which it is known that for orientations within this range the sensor readings can be reliably approximated as a function of the deviation of the actual orientation of the air pollutant sensor device from its optimal orientation.

In order to further improve the accuracy of the correction applied to an actual air pollutant count obtained with the air pollutant sensor in the air pollutant sensor device, the actual air pollutant count may be obtained by the air pollutant sensor over a defined time interval and the processor may receive a plurality of further sensor signals during said defined time interval, wherein the processor is adapted to determine the orientation of the portable air pollutant sensor device as an average orientation of the portable air pollutant sensor device during said defined time interval from the received plurality of further sensor signals.

Embodiments of the present invention are not limited to obtaining single air pollutant measurements. In a further embodiment, the processor is adapted to track changes in the ambient air pollutant concentration over a monitoring period from a plurality of sensor signals received from the air pollutant sensor of a portable air pollutant sensor device during said monitoring period such that trends in the concentration of the air pollutant can be obtained over the monitoring period. This for example may provide valuable information about certain periods of the day during which the ambient air pollutant concentration has a particular characteristic values, e.g. characteristically low or high values, which information may be leveraged by the user of the device, for example to determine the best time of day to exercise whilst limiting exposure to the ambient air pollutant of interest.

During such a monitoring period, the orientation of the air pollutant sensor device may temporarily be outside the range of orientations for which the ambient air pollutant concentration can be reliably determined, i.e. at least one of the plurality of sensor signals indicating the determined orientation of the portable air pollutant sensor device lies outside the defined range of orientations. In such a scenario, the processor may be adapted to extrapolate the ambient air pollutant concentration for the at least one of plurality of sensor signals from a first sensor signal and a second sensor signal of the plurality of sensor signals for which the determined orientation of the portable air pollutant sensor device lies inside the defined range of orientations, the first sensor signal preceding the at least one of the plurality of sensor signals and the second sensor signal following the at least one of the plurality of sensor signals to facilitate an estimation of the ambient air pollutant concentration at the point in time at which the at least one of the plurality of sensor signals indicating the determined orientation of the portable air pollutant sensor device lying outside the defined range of orientations is generated. This ensures that an estimate of the ambient air pollutant concentration is provided for each interval of the monitoring period even if during such an interval the orientation of the portable air pollutant sensor device lies outside the acceptable range of orientations.

Where during such a monitoring period at least some of the ambient air pollutant concentrations are estimated in this manner, the processor may be further adapted to calculate an indication of the reliability of the monitored ambient air pollutant concentration over the monitoring period based on a number of sensor signals for which the determined orientation of the portable air pollutant sensor device lies outside the defined range of orientations during said monitoring period such that a user of the device may be presented with an indication of accuracy of the ambient air pollutant concentrations collected over the monitoring period.

The device may further comprise an output device, wherein the processor further is adapted to generate a control signal indicative of the determined ambient air pollutant concentration for the output device to present a user of the device with the determined ambient air pollutant concentration.

The device may further comprise an output device, wherein the processor further is adapted to generate a control signal indicative of the determined orientation of the portable air pollutant sensor device for the output device. These respective output devices may be a single device, e.g. a display or the like, or may be different devices. The processor further may be adapted to generate a control signal indicative of a change in the determined orientation of the portable air pollutant sensor device for the output device over a time period, which information for example may be used by the user of the device to learn how to correctly use, i.e. position, the portable air pollutant sensor device, e.g. to minimize the number of occasions during which the orientation of the portable air pollutant sensor device is outside the accepted range of orientations when attempting to obtain an ambient air pollutant concentration.

The device including the processor may be separate to the portable air pollutant sensor device, for example when the portable air pollutant sensor device is a wearable a pollutant sensor device. For example, the device including the processor may be a computing device, e.g. a portable computing device such as a smart watch, smart phone or a tablet or the like, a laptop computer, a desktop computer, an Internet service on a network server, and so on, in which case the device including the processor may be communicatively coupled to the portable air pollutant sensor device over a communication link such as a wireless or wired communication link. Alternatively, the device including the processor may further comprise the portable air pollutant sensor device, thereby providing a self-contained portable air pollutant sensor device in the sense that the device itself also performs the processing of its sensor signals. Such a portable air pollutant sensor device may be integrated in a portable computing device such as a tablet or smart phone or alternatively may be a wearable air pollutant sensor device including the processor functionality.

In the case of such an integrated portable air pollutant sensor device, the device may further comprise the orientation sensor. Alternatively, the orientation sensor may be provided separately to the air pollutant sensor device and may be attached to the air pollutant sensor device in any suitable manner in order to ensure a defined relationship between the orientation of the orientation sensor and the portable air pollutant sensor device.

In example embodiments, the ambient air pollutant sensor is a particulate matter sensor such as a PM 2.5 sensor although it should be understood that embodiments of the present invention are not limited to particulate matter sensors and alternatively may be any type of sensor for determining the concentration of an ambient air pollutant of interest, e.g. a volatile organic compounds sensor such as a formaldehyde sensor or toluene sensor, and so on.

According to another aspect, there is provided a computer program product comprising a computer readable storage medium having computer readable program instructions embodied therewith for, when executed on the processor of the device according to embodiments of the present invention cause the processor to receive a sensor signal from an air pollutant sensor of a portable air pollutant sensor device comprising an air flow channel extending between a first opening open to an ambient environment including the ambient air pollutant concentration and a second opening open to said ambient environment, the air flow channel comprising a sensing area including the air pollutant sensor and a heating element for inducing an air flow through said air flow channel; receive a further sensor signal from an orientation sensor arranged to determine an orientation of the portable air pollutant sensor device; determine an orientation of the portable air pollutant sensor device from the received further sensor signal; compare the determined orientation of the portable air pollutant sensor with a defined range of orientations including an optimal orientation of the portable air pollutant sensor device for sensing said ambient air pollutant concentration; and determine the ambient air pollutant concentration only if the determined orientation of the portable air pollutant sensor lies within the defined range of orientations by determining an actual air pollutant count from the received sensor signal and correcting the actual air pollutant count based on the determined orientation of the portable air pollutant sensor if the determined orientation differs from the optimal orientation. Such a computer program product may be used to configure any of the previously mentioned computing devices to process the sensor signals of the portable air pollutant sensor device as previously explained.

The actual air pollutant count may be obtained by the air pollutant sensor over a defined time interval and the processor may receive a plurality of further sensor signals during said defined time interval, wherein the computer readable program instructions further cause the processor to determine the orientation of the portable air pollutant sensor device as an average orientation of the portable air pollutant sensor device during said defined time interval from the received plurality of further sensor signals to further improve the accuracy of the ambient air pollutant concentration calculated by the processor.

The computer readable program instructions may cause the processor to track changes in the ambient air pollutant concentration over a monitoring period from a plurality of sensor signals received from the air pollutant sensor of a portable air pollutant sensor device during said monitoring period in order to configure the processor to monitor changes in the ambient air pollutant concentration during the monitoring period.

In case for at least one of the plurality of sensor signals the determined orientation of the portable air pollutant sensor device lies outside the defined range of orientations, the computer readable program instructions may cause the processor to extrapolate the ambient air pollutant concentration for the at least one of plurality of sensor signals from a first sensor signal and a second sensor signal of the plurality of sensor signals for which the determined orientation of the portable air pollutant sensor device lies inside the defined range of orientations, the first sensor signal preceding the at least one of the plurality of sensor signals and the second sensor signal following the at least one of the plurality of sensor signals such that an estimate of the ambient air pollutant concentration over the full monitoring period can be provided even when some of the sensor signals obtained during the monitoring period originated from a portable air pollutant sensor device in an orientation outside the accepted range of orientations as previously explained.

In such a scenario, the computer readable program instructions may cause the processor to calculate an indication of the reliability of the monitored ambient air pollutant concentration over the monitoring period based on a number of sensor signals for which the determined orientation of the portable air pollutant sensor device lies outside the defined range of orientations during said monitoring period such that a user may be presented with this indication of the reliability, e.g. through an output device of the device including the processor.

BRIEF DESCRIPTION OF THE DRAWINGS

Embodiments of the invention are described in more detail and by way of non-limiting examples with reference to the accompanying drawings, wherein:

FIG. 1 schematically depicts an ambient air pollutant sensor device according to an embodiment;
FIG. 2 schematically depicts an actual air flow rate through such an ambient air pollutant sensor device as a function of its orientation;
FIG. 3 schematically depicts a range of orientation angles of such an ambient air pollutant sensor device in which it can be accurately operated;
FIG. 4 is a flow chart of an example operation method of such an ambient air pollutant sensor device;
FIG. 5 is a flow chart of another example operation method of such an ambient air pollutant sensor device;
FIG. 6 schematically depicts an ambient air pollutant sensor device according to another embodiment; and
FIG. 7 schematically depicts a device arrangement including an ambient air pollutant sensor device according to yet another embodiment.

DETAILED DESCRIPTION OF THE EMBODIMENTS

It should be understood that the Figures are merely schematic and are not drawn to scale. It should also be understood that the same reference numerals are used throughout the Figures to indicate the same or similar parts.

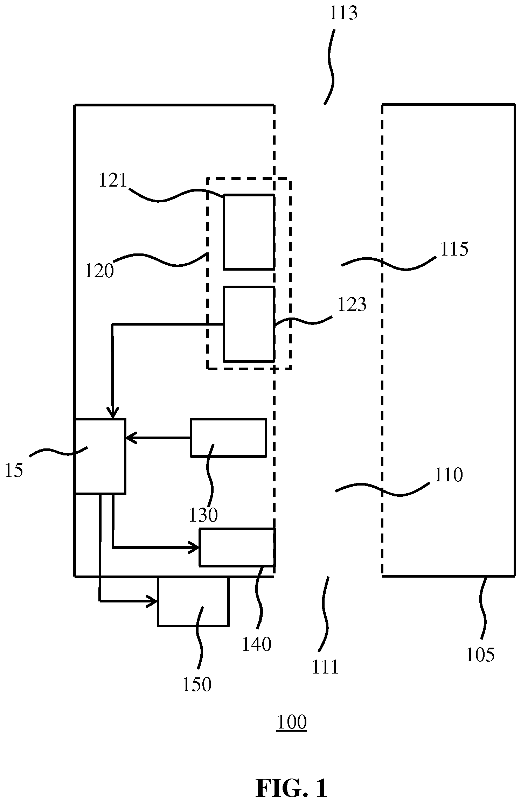

FIG. 1 schematically depicts a portable ambient air pollutant sensor device 100 according to an embodiment. The ambient air pollutant sensor device 100 comprises an air flow channel 110 extending through a housing 105 of the ambient air pollutant sensor device 100. The housing 105 may be made of any suitable material, e.g. a plastics material, a metal, a metal alloy or combinations thereof. The air flow channel 110 has a first opening 111 and a second opening 113 that are each open to an ambient environment. For example, the first opening 111 may oppose the second opening 113.

In the flow channel 110 in between the first opening 111 and a second opening 113 is located a sensing area 115 housing an ambient air pollutant sensor 120 for detecting an ambient air pollutant flowing through the air flow channel 110. In the context of the present application, an ambient air pollutant is a pollutant of the ambient air, i.e. the atmospheric air in which the portable air pollutant sensor device 100 operates. In an example embodiment, the ambient air pollutant sensor 120 is a particulate matter (particle) sensor although embodiments of the present invention are not limited thereto. Such a particle sensor for example may be a PM2.5 sensor although other particle sensors, e.g. sensors adapted to (also) detect other sized particles such as ultrafine particles (UFP), PM5.0 or PM10.0 particles, may also be considered.

In an embodiment, the particle sensor is an optical particle sensor comprising an optical module 121 for emitting light through the sensing area and a detector 123 for detecting light scattered by particles in the air flowing through the sensing area 115. The optical module 121 for example may be a LED module or a laser module and the detector 123 for example may be a photodetector such as a photodiode or the like. Any suitable optical particle sensor design may be used, as such optical particle sensors are well-known per se. One known principle is a LED based sensor as for example disclosed in JP2008145353A and CN104089858A. Another known principle is a laser-based sensor, for example, a laser-based PM2.5 detector used to detect atmospheric particles having a size (diameter) of less than 2.5 $\mu$m, as for example disclosed in CN203551443U, CN203824872U and CN105021501A. As such optical particle sensor designs are commonplace, they will not be explained in further detail for the sake of brevity.

As an alternative to such particle sensors, the ambient air pollutant sensor 120 may be a VOC sensor or the like, or more generally, may be any sensor for determining the concentration of an ambient air pollutant of interest.

The portable ambient air pollutant sensor device 100 further comprises an orientation sensor 130 such a gyroscope or the like to determine the orientation of the portable ambient air pollutant sensor device 100, in particular the actual orientation of the air flow channel 110 relative to a vertical orientation. Preferably, this includes the generation of orientation sensor signal indicative of the orientation of the portable ambient air pollutant sensor device 100 in two dimensions, e.g. in the XZ plane and in the XY plane.

In addition, the portable ambient air pollutant sensor device 100 comprises a heating element 140 in the air flow channel 110 for inducing an air flow through the air flow channel 110. Such a heating element 140 for example may be a resistive heating element, e.g. a resistor or the like, which generates a defined amount of heat in response to being provided with a defined electrical current. Given that the dimensions, e.g. volume, of the air flow channel 110 are well-defined, the defined amount of heat consequently generates a defined air flow speed through the air flow channel 110 when the air flow channel 110 is in a defined orientation, which will also be referred to as an optimal orientation.

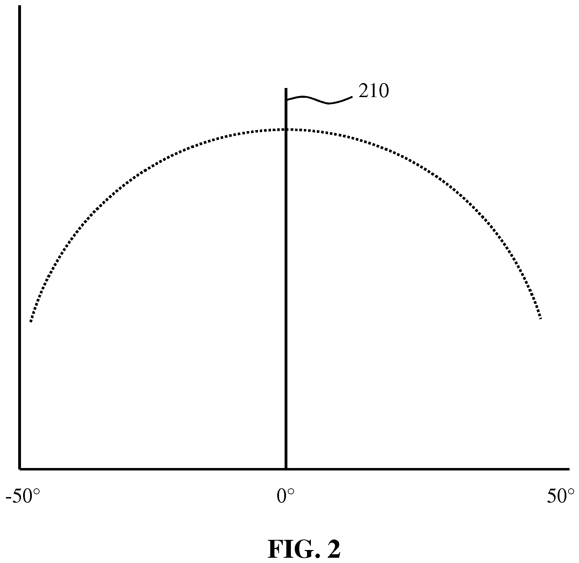

Embodiments of the present invention are based on the insight that where the orientation of the portable ambient air pollutant sensor device 100 deviates from this optimal orientation, the actual air flow speed through the air flow channel 110, and in particular through the sensing area 115, deviates from the defined air flow speed with the portable ambient air pollutant sensor device 100 in its optimal orientation. This is schematically depicted in FIG. 2, which depicts the air flow speed (Y-axis) induced by the heating element 143 air flow channel 110 as a function of the orientation (X-axis) of the portable ambient air pollutant sensor device 100, in which 0° symbolizes the optimal orientation angle of the portable ambient air pollutant sensor device 100, e.g. a vertical orientation. As can be seen from FIG. 2, with increasing deviation of the orientation angle of the portable ambient air pollutant sensor device 100 from its optimal orientation angle, the actual air flow speed through the air flow channel 110 decreases, with the rate of decrease increasing for increasingly large deviations from the optimal orientation angle. It should however be understood that this is by way of non-limiting example only, as for some designs of the air flow channel 110 certain deviations from the optimal orientation of the portable ambient air pollutant sensor device 100 can lead to an increase rather than decrease in the air flow speed through the air flow channel 110 as induced by the heating element 140. It is furthermore noted that as explained previously, such deviations may be seen as increases in the uncertainty of an approximated actual air flow speed through the air flow channel 110, as the actual air flow speed may depend from a number of factors such as orientation of the portable ambient air pollutant sensor device 100 and temperature of the ambient air, such that the actual air flow speed through the air flow channel 110 may be assumed to lie within a range of air flow speeds, which range is defined based on the variance in these factors. Where such uncertainty becomes too large, the approximated actual air flow speed through the air flow channel 110, e.g. through the sensing area 115, may no longer be reliably assumed to lie within such a range, such that the ambient air pollutant levels may no longer be reliably approximated.

It is noted for the avoidance of doubt that FIG. 2 depicts a deviation in a single plane for the sake of clarity, whereas in practice deviations in two planes, e.g. the XZ-plane and the XY-plane typically will be considered. Therefore, where reference is made in the remainder of this application the determination of the actual orientation of the portable ambient air pollutant sensor device 100 using its orientation sensor 130, it should be understood that this preferably includes the determination of a deviation in the orientation of the portable ambient air pollutant sensor device 100 from its optimal orientation in both these planes.

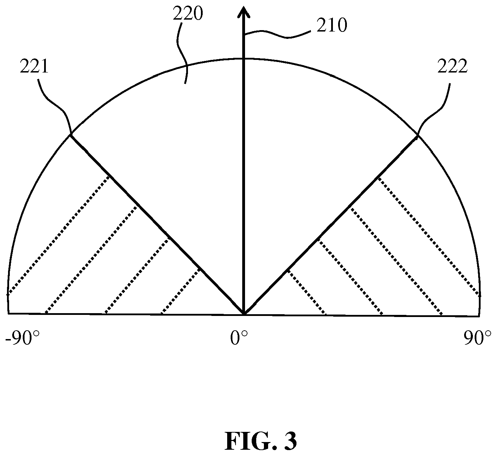

In order to factor in the effect of the orientation of the portable ambient air pollutant sensor device 100 on the air flow speed through the air flow channel 110, the portable ambient air pollutant sensor device 100 further comprises a processor 15 communicatively coupled to the ambient air pollutant sensor 120, e.g. to the detector 123 of a particle sensor and to the orientation sensor 130. The processor 15 is arranged to derive an ambient air pollutant concentration in the air flow through the air flow channel 110 from one or more signals received from the ambient air pollutant sensor 120 only if it determines based on sensor signals obtained from the orientation sensor 130 that the portable ambient air pollutant sensor device 100 lies within a defined range 220 of orientations as schematically depicted in FIG. 3, The defined range 220 of orientations, i.e. orientation angles including the optimal orientation angle 210 of the portable ambient air pollutant sensor device 100 typically includes a first end value 221 and a second end value 222 on opposite sides of the optimal value 210 of the orientation angle of the portable ambient air pollutant sensor device 100. As will be understood by the skilled person, the chosen and values 221 and 222 typically are a function of the design (shape) of the air flow channel 110.

As previously explained, such a defined range 220 of orientations of the portable ambient air pollutant sensor device 100 defines the acceptable orientation angles of the portable ambient air pollutant sensor device 100 for which the air flow speed through the air flow channel 110 can be accurately determined, as outside the range 220 small variations in the orientation of the portable ambient air pollutant sensor device 100 lead to substantial changes in the air flow speed through its air flow channel 110. As will be understood from the foregoing, such a defined range 220 of acceptable orientations of the portable ambient air pollutant sensor device 100 may be defined for each of the two dimensions, e.g. for the XZ-plane and XY-plane as determined with the orientation sensor 130, which respective ranges of acceptable orientations may be different for the two dimensions. In an embodiment, the processor 15 is configured to only determine the concentration of the ambient air pollutants from the sensor signals received from the ambient air pollutant sensor 120 if the orientation of the portable ambient air pollutant sensor device 100 is considered acceptable in both these dimensions, i.e. the orientation of the portable ambient air pollutant sensor device 100 lies within each of these respective ranges.

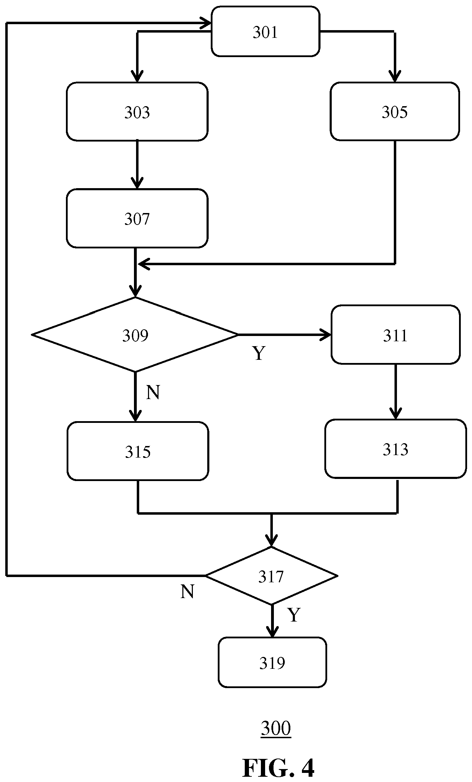

The operation of the portable ambient air pollutant sensor device 100 and its processor 15 will be explained in more detail with the aid of FIG. 4, which depicts a flowchart of an operation mode 300 of the portable ambient air pollutant sensor device 100. In operation 301, the operation mode 300 is initiated, e.g. by starting up the portable ambient air pollutant sensor device 100, after which the processor 15 receives one or more orientation sensor signals from the orientation sensor 130 in operation 303 and one or more ambient air pollutant sensor signals from the ambient air pollutant sensor 120 in operation 305. The operations 303 and 305 are typically performed in parallel as the orientation sensor signals are relevant to the assessment of whether the ambient air pollutant sensor signals are reliable enough for the determination of an ambient air pollutant concentration from these signals.

To this end, the processor 15 in operation 307 determines the orientation of the portable ambient air pollutant sensor device 100, e.g. its orientation in the aforementioned two dimensions, from the received orientation sensor signals and determines in operation 309 whether the orientation of the portable ambient air pollutant sensor device 100 lies within the aforementioned one or more ranges 220 of acceptable orientation angles for the portable ambient air pollutant sensor device 100. If this is the case, the processor 15 proceeds to operation 311 in which the concentration of the ambient air pollutant is determined from the one or more ambient air pollutant sensor signals received from the ambient air pollutant sensor 120 in operation 305. If necessary, the thus calculated concentration of the ambient air pollutant is corrected using the actual orientation of the portable ambient air pollutant sensor device 100 determined in operation 307, i.e. when the orientation of the portable ambient air pollutant sensor device 100 deviates from its optimal orientation. For example, the processor 15 may be configured to correct the measured concentration P of the ambient air pollutant using the following formula:

$$P' = P/\{(1-f1)*(1-f2)\}$$

In this formula, f1 and f2 are correction factors for the flow rate through the air flow channel 110 for one of the two dimensions in which the orientation of the portable ambient air pollutant sensor device 100 is determined. The correction factors f1 and f2 are variables that are function of the actual orientation of the portable ambient air pollutant sensor device 100 in the associated dimension. The correction factors f1 and f2 may be determined in any suitable manner, e.g. empirically determined as will be readily understood by the skilled person. The resulting corrected ambient air pollutant concentration P' thus provides a more accurate estimate of the real ambient air pollutant concentration.

At this point, it is noted that the rate of the generation of sensor signals by the ambient air pollutant sensor 120 and the orientation sensor 130 may not be identical. For example, the orientation sensor 130 may generate orientation sensor signals at a higher rate than the ambient air pollutant sensor 120 generates ambient air pollutant sensor signals, such that for each ambient air pollutant sensor signal generated in operation 305 multiple orientation sensor signals may have been generated in operation 303. In such a scenario, the processor 15 may be configured to determine an average orientation of the portable ambient air pollutant sensor device 100 in operation 307 from the multitude of orientation sensor signals received in operation 303 and to base the evaluation of the validity of the orientation of the portable ambient air pollutant sensor device 100 on its determined average orientation.

In operation 313, the processor 15 may generate a control signal to control an output device 150 of the portable ambient air pollutant sensor device 100 such that the output device 150 produces an output indicative of the corrected ambient air pollutant concentration P' such that a user can learn the value of the ambient air pollutant concentration from the output device 150. Such an output device 150 may be any suitable output device, e.g. a display, loudspeaker, a light source or the like, or a combination of such output devices. Upon generation of the control signal for the output device 150, it is checked by the processor 15 if the operation mode 300 is to be continued, i.e. if another ambient air pollutant concentration is to be determined, in which case the operating mode 300 reverts back to 301 such that the next set of orientation sensor signals and ambient air pollutant sensor signals can be acquired in concurrent operations 303 and 305. Otherwise, this operating mode 300 of the processor 15 terminates in 319.

At this point it is noted that the output device 150 not necessarily forms part of the portable ambient air pollutant sensor device 100. In an alternative embodiment the output device 150 forms part of an external device (not shown) that is communicatively coupled to the portable ambient air pollutant sensor device 100, e.g. over a wired or wireless communication link, in which case the portable ambient air pollutant sensor device 100 typically comprises a communication module such as a wired or wireless communication module to establish the communication between the processor 15 of the portable ambient air pollutant sensor device 100 and the external device. Such an external device itself may be a portable device such as a smart phone or a tablet and may be configured, e.g. using a dedicated software application (app) to generate the output on its output device 150 such that a user can obtain real-time feedback through the external device about how the portable ambient air pollutant sensor device 100 is to be used. This for example is particularly useful where the portable ambient air pollutant sensor device 100 is a wearable sensor device, which wearable devices are typically compact devices without output devices such that communication between the portable ambient air pollutant sensor device 100, e.g. wireless communication using Bluetooth or the like, provides such a wearable sensor device with output capability to the external device.

If on the other hand it is determined by the processor 15 in operation 309 that the (average) orientation of the portable ambient air pollutant sensor device 100 lies outside at least one of the defined ranges 220 of acceptable orientation angles for the portable ambient air pollutant sensor device 100, the processor 15 may proceed to operation 315 in which the processor 15 generates a warning signal trigger, which warning signal may trigger an output device such as the output device 150 or another output device of the portable ambient air pollutant sensor device 100 to generate a warning signal that warms the user of the portable ambient air pollutant sensor device 100 that the portable ambient air pollutant sensor device 100 is held in an orientation in which the processor 15 cannot reliably determine the actual ambient air pollutant concentration from the ambient air pollutant sensor signals received in operation 303, which may trigger a user to adjust the orientation of the portable ambient air pollutant sensor device 100 accordingly, such that it may be decided in operation 317 to acquire another set of orientation sensor signals and ambient air pollutant sensor signals as previously explained from which an accurate determination of the ambient air pollutant concentration can be derived. In an embodiment in which the portable ambient air pollutant sensor device 100 is a wearable sensor device, such a warning signal for example can indicate to its user that the device is incorrectly attached to the user's body, e.g. arm or leg, such as too insecurely attached, such that the user can rectify this, e.g. tighten a strap of the wearable sensor device or the like, to reduce the likelihood of the portable ambient air pollutant sensor device 100 being in an orientation outside its acceptable ranges of orientation angles.

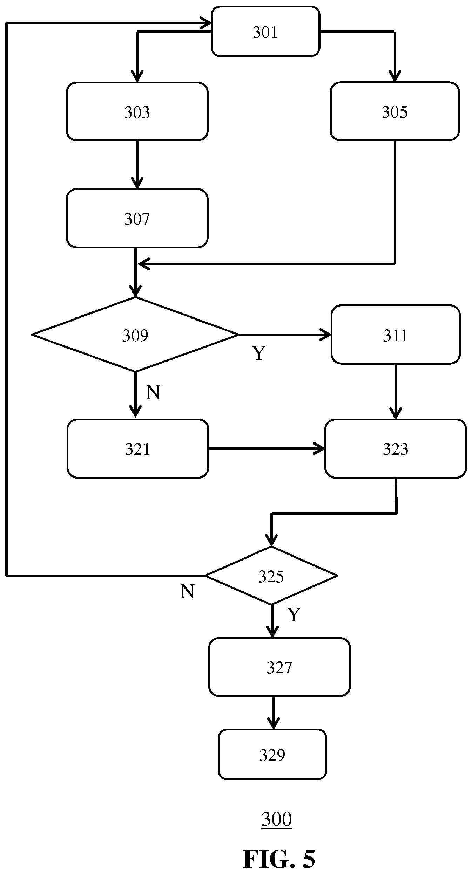

Operation mode 300 of the processor 15 may be deployed to obtain single measurements of the ambient air pollutant concentration with the portable ambient air pollutant sensor device 100. However, in an embodiment the processor 15 is further configured to monitor the ambient air pollutant concentration over a period of time (i.e. a monitoring period), e.g. to monitor changes or trends in the ambient air pollutant concentration. Such an extended operation mode 300 is depicted by the flowchart of FIG. 5, and differs from the operation mode 300 in FIG. 4 that during the monitoring period the processor 15 typically assesses a plurality of sets of orientation sensor signals from operation 303 and ambient air pollutant sensor signals from operation 305 at different points in time of the monitoring period. For each set of orientation sensor signals and accompanying ambient air pollutant sensor signals for a given point in time during the monitoring period it is determined by the processor 15 in operation 309 whether the orientation of the portable ambient air pollutant sensor device 100 is acceptable for the purpose of the determination of an accurate ambient air pollutant concentration from the ambient air pollutant sensor signals as previously explained, which if this is the case causes the processor 15 to determine the ambient air pollutant concentration in operation 311 as previously described, which determined ambient air pollutant concentration subsequently may be stored in a data storage device (not shown) communicatively coupled to the processor 15, e.g. a data storage device such as a memory device, solid state disk, hard disk and so on of the device comprising the processor 15. Alternatively, the data storage device may be a network-attached data storage device to which the processor 15 has access, e.g. through a network interface of the device comprising a processor 15. Such a network-attached data storage device may be a data storage arrangement accessible through the Internet, e.g. a cloud storage arrangement.

If on the other hand the orientation of the portable ambient air pollutant sensor device 100 lies outside acceptable ranges for the purpose of the determination of an accurate ambient air pollutant concentration from the ambient air pollutant sensor signals as previously explained, the processor 15 may proceed to operation 321 in which the point in time of the monitoring period is marked 'invalid' or the like to indicate the orientation of the portable ambient air pollutant sensor device 100 lying outside acceptable ranges, and the data stored in operation 323 may be updated accordingly.

However, in an alternative embodiment the processor 15 extrapolates an expectation value of the ambient air pollutant concentration at this point in time from an earlier determined ambient air pollutant concentration and a later determined ambient air pollutant concentration, e.g. the closest determinations in time for which the orientation of the portable ambient air pollutant sensor device 100 was within acceptable ranges. Such an extrapolation may be performed by the processor 15 as soon as the next ambient air pollutant concentration can be determined owing to the fact that the orientation of the portable ambient air pollutant sensor device 100 has returned to within acceptable ranges or alternatively may be performed upon completion of the monitoring period as determined in operation 325.

In a further refinement, such an extrapolated value may be labelled as unreliable in certain scenarios. For example, if the orientation of the portable ambient air pollutant sensor device 100 lies outside its acceptable parameters for a relatively short period in time, it is likely that the extrapolation of the ambient air pollutant concentration is reliable, such that such labelling is not required. Similarly, where the orientation of the portable ambient air pollutant sensor device 100 lies outside its acceptable parameters for a longer period of time but the difference between the determined ambient air pollutant concentrations used for the extrapolation is small, it is again likely that the extrapolated value is reliable such that such labelling is not required. However, where the orientation of the portable ambient air pollutant sensor device 100 lies outside its acceptable parameters for a relatively short period and a large difference between the determined ambient air pollutant concentrations used for the extrapolation is observed, the extrapolated value may be labelled as unreliable such that when an output is produced of the determined or extrapolated ambient air pollutant concentrations, e.g. on a display device, unreliable extrapolated values can be readily recognized by the user from the attached labels. In the context of the present location, such labels may be generated in any suitable manner, for example by using colour coding to differentiate between reliable and unreliable ambient air pollutant sensor concentrations.

Upon termination of the monitoring period, the processor 15 may proceed to operation 327 in which the ambient air pollutant concentrations obtained during the monitoring period are evaluated, e.g. to determine trends or the like in the concentration data, which evaluation may be generated as an output on the output device 150 as previously described. In an embodiment, this evaluation further includes the determination of the reliability of the monitoring data, in particular where the monitoring data includes extrapolated values of the ambient air pollutant concentration for points in time during the monitoring period for which the orientation of the portable ambient air pollutant sensor device 100 was outside the aforementioned acceptable ranges of its orientation angles. This for example may be achieved by the processor 15 calculating an indication of the reliability of the monitored ambient air pollutant concentration over the monitoring period based on a number of extrapolated ambient air pollutant concentrations, i.e. the number of ambient air pollutant sensor signals for which the determined orientation of the portable air pollutant sensor device 100 was outside at least one of the defined ranges of orientations. The processed 15 may be further configured to generate an output signal for controlling the output device 150 or another output device of the portable ambient air pollutant sensor device 100 to generate an output of the determined indication of the reliability of the output device 150. The extended operation mode 300 subsequently may terminate in 329.

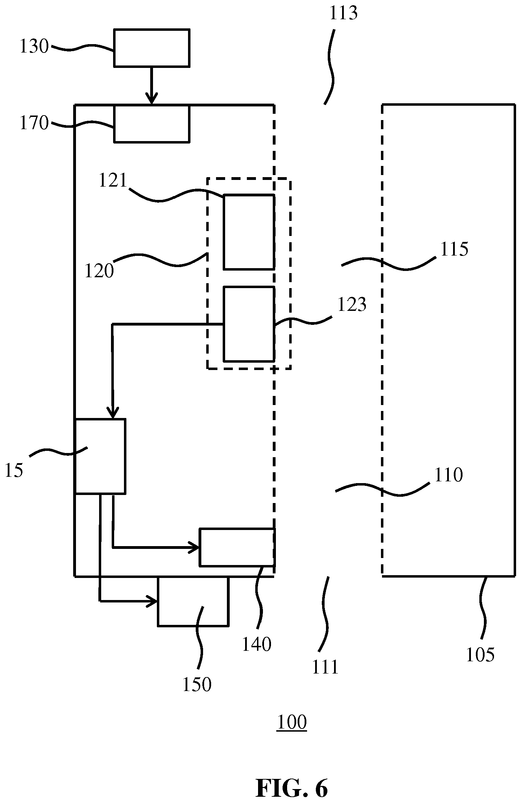

In the above embodiments of the portable ambient air pollutant sensor device 100, the orientation sensor 130 forms an integral part of the portable ambient air pollutant sensor device 100. However, FIG. 6 schematically depicts an alternative embodiment in which a separate orientation sensor 130 is used to determine the orientation of the portable ambient air pollutant sensor device 100. In this embodiment, the separate orientation sensor 130 may be arranged to communicate with the portable ambient air pollutant sensor device 100 to a communication module 170 of the portable ambient air pollutant sensor device 100, which communication module 170 is communicatively coupled to the processor 15 such that the orientation sensor signals generated with the separate orientation sensor 130 can be passed onto the processor 15. Such a communication module 170 may be a wireless communication module employing any suitable wireless communication protocol, e.g. Wi-Fi, Bluetooth, NFC, and so on or alternatively may be a wired communication module to which the separate orientation sensor 130 is connected through one or more wires. The separate orientation sensor 130 may be physically coupled to the housing 105 of the portable ambient air pollutant sensor device 100 to ensure a fixed relationship between the orientation of the separate orientation sensor 130 and the portable ambient air pollutant sensor device 100. Such a physical coupling may be achieved in any suitable manner, e.g. through a mating arrangement such as a tongue and groove arrangement or the like.

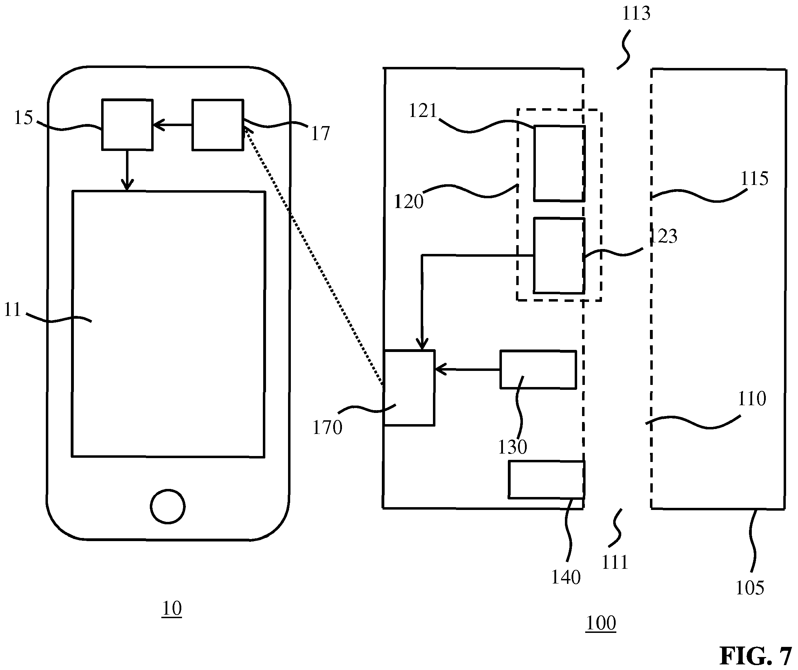

In yet another embodiment of the present invention as schematically depicted in FIG. 7, the processor 15 is located in a computing device 10, which for example may be a portable computing device such as a smart phone, tablet computer, laptop computer or the like, or alternatively may be a desktop computer or the like. The computing device 10 further comprises a communication module 17 communicatively coupled to the processor 15, e.g. a wireless communication module adapted to wirelessly communicate with the previously described wireless communication module 170 of the portable ambient air pollutant sensor device 100 such that the processor 15 can receive the orientation sensor signals from the orientation sensor 130 and the ambient air pollutant sensor signals from the ambient air pollutant sensor 120 over the wireless communication link between the wireless communication module 170 of the portable ambient air pollutant sensor device 100 and the wireless communication module 17 of the computing device 10. The computing device 10 may further comprise an output device 11 under control of the processor 15, e.g. a display device or the like, onto which the processor 15 may generate outputs relating to the respective sensor signals received from the portable ambient air pollutant sensor device 100 as previously described.

In this embodiment, the portable ambient air pollutant sensor device 100 may further comprise a microcontroller or the like (not shown), e.g. for controlling the ambient air pollutant sensor 120, the orientation sensor 130, the heating element 140 and the wireless communication module 170. The embodiment of FIG. 7 for example is particularly useful where the portable ambient air pollutant sensor device 100 is a wearable ambient air pollutant sensor device 100 such that processing of the sensor signals generated with the device can be processed at another device, i.e. the computing device 10, thereby reducing the form factor of the ambient air pollutant sensor device 100 owing to the fact that the processor 15 can be omitted from the design of the ambient air pollutant sensor device 100.

In the above embodiments, the processor 15 may be configured to perform the various operations, e.g. the (extended) operation mode 300 as schematically depicted in FIG. 4 and FIG. 5, using a computer program product comprising a computer readable storage medium having computer readable program instructions embodied therewith for, when executed on the processor 15 cause the processor 15 to perform these operations. Any suitable computer readable storage medium may be used for this purpose, such as for example an optically readable medium such as a CD, DVD or Blu-Ray disc, a magnetically readable medium such as a hard disk, an electronic data storage device such as a memory stick or the like, and so on. The computer readable storage medium may be a medium that is accessible over a network such as the Internet, such that the computer readable program instructions may be accessed over the network. For example, the computer readable storage medium may be a network-attached storage device, a storage area network, cloud storage or the like. The computer readable storage medium may be an Internet-accessible service from which the computer readable program instructions may be obtained. In an embodiment, the processor 15 is adapted to retrieve the computer readable program instructions from such a computer readable storage medium and to create a new computer readable storage medium by storing the retrieved computer readable program instructions in a data storage arrangement (not shown), e.g. in a memory device or the like forming part of the data storage arrangement.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A device (10, 100) for determining an ambient air pollutant concentration, the device comprising a processor (15) adapted to:

   receive a sensor signal from an air pollutant sensor (120) of a portable air pollutant sensor device (100) comprising an air flow channel (110) extending between a first opening (111) open to an ambient environment including the ambient air pollutant concentration and a second opening (113) open to said ambient environment, the air flow channel comprising a sensing area (115) including the air pollutant sensor (120) and a heating element (140) for inducing an air flow through said air flow channel;
   receive a further sensor signal from an orientation sensor (130) arranged to determine an orientation of the portable air pollutant sensor device;
   determine an orientation of the portable air pollutant sensor device from the received further sensor signal;
   compare the determined orientation of the portable air pollutant sensor with a defined range of orientations (220) including an optimal orientation (210) of the portable air pollutant sensor device for sensing said ambient air pollutant concentration;
   determine the ambient air pollutant concentration only if the determined orientation of the portable air pollutant sensor lies within the defined range of orientations by determining an actual air pollutant count from the received sensor signal and correcting the actual air pollutant count based on the determined orientation of the portable air pollutant sensor if the determined orientation differs from the optimal orientation.

2. The device (10, 100) of claim 1, wherein the actual air pollutant count is obtained by the air pollutant sensor (120) over a defined time interval and the processor (15) receives a plurality of further sensor signals during said defined time interval, wherein the processor is adapted to determine the orientation of the portable air pollutant sensor device (100) as an average orientation of the portable air pollutant sensor device during said defined time interval from the received plurality of further sensor signals.

3. The device (10, 100) of claim 1, wherein the processor (15) is adapted to track changes in the ambient air pollutant concentration over a monitoring period from a plurality of sensor signals received from the air pollutant sensor (120) of a portable air pollutant sensor device (100) during said monitoring period.

4. The device (10, 100) of claim 3, wherein for at least one of the plurality of sensor signals the determined orientation of the portable air pollutant sensor device (100) lies outside the defined range of orientations (220), and wherein the processor (15) is adapted to extrapolate the ambient air pollutant concentration for the at least one of plurality of sensor signals from a first sensor signal and a second sensor signal of the plurality of sensor signals for which the determined orientation of the portable air pollutant sensor device lies inside the defined range of orientations, the first sensor signal preceding the at least one of the plurality of sensor signals and the second sensor signal following the at least one of the plurality of sensor signals.

5. The device (10, 100) of claim 4, wherein the processor (15) further is adapted to calculate an indication of the reliability of the monitored ambient air pollutant concentration over the monitoring period based on a number of sensor signals for which the determined orientation of the portable air pollutant sensor device (100) lies outside the defined range of orientations during said monitoring period.

6. The device (10, 100) of claim 1, further comprising an output device (150), wherein the processor (15) further is adapted to generate a control signal indicative of the determined ambient air pollutant concentration for the output device.

7. The device (10, 100) of claim 1, further comprising an output device (150), wherein the processor (15) further is adapted to generate a control signal indicative of the determined orientation of the portable air pollutant sensor device (100) for the output device.

8. The device (10, 100) of claim 7, wherein the processor (15) further is adapted to generate a control signal indicative of a change in the determined orientation of the portable air pollutant sensor device (100) for the output device over a time period.

9. The device of claim 1, further comprising the portable air pollutant sensor device (100).

10. The device (10, 100) of claim 9, further comprising the orientation sensor (130).

11. A computer program product comprising a computer readable storage medium having computer readable program instructions embodied therewith for, when executed on the processor (15) of the device of claim 1, cause the processor to:

receive a sensor signal from an air pollutant sensor (120) of a portable air pollutant sensor device (100) comprising an air flow channel (110) extending between a first opening (111) open to an ambient environment including the ambient air pollutant concentration and a second opening (113) open to said ambient environment, the air flow channel comprising a sensing area (115) including the air pollutant sensor (120) and a heating element for inducing an air flow through said air flow channel;
receive a further sensor signal from an orientation sensor arranged to determine an orientation of the portable air pollutant sensor device;
determine an orientation of the portable air pollutant sensor device from the received further sensor signal;
compare the determined orientation of the portable air pollutant sensor with a defined range of orientations (220) including an optimal orientation (210) of the portable air pollutant sensor device for sensing said ambient air pollutant concentration; and
determine the ambient air pollutant concentration only if the determined orientation of the portable air pollutant sensor lies within the defined range of orientations by determining an actual air pollutant count from the received sensor signal and correcting the actual air pollutant count based on the determined orientation of the portable air pollutant sensor if the determined orientation differs from the optimal orientation.

12. The computer program product of claim 11, wherein the actual air pollutant count is obtained by the air pollutant sensor over a defined time interval and the processor receives a plurality of further sensor signals during said defined time interval, wherein the computer readable program instructions further cause the processor to determine the orientation of the portable air pollutant sensor device as an average orientation of the portable air pollutant sensor device during said defined time interval from the received plurality of further sensor signals.

13. The computer program product of claim 11, wherein the computer readable program instructions cause the processor to track changes in the ambient air pollutant concentration over a monitoring period from a plurality of sensor signals received from the air pollutant sensor (120) of a portable air pollutant sensor device (100) during said monitoring period.

14. The computer program product of claim 13, wherein for at least one of the plurality of sensor signals the determined orientation of the portable air pollutant sensor device lies outside the defined range of orientations, and wherein the computer readable program instructions cause the processor to extrapolate the ambient air pollutant concentration for the at least one of plurality of sensor signals from a first sensor signal and a second sensor signal of the plurality of sensor signals for which the determined orientation of the portable air pollutant sensor device lies inside the defined range of orientations, the first sensor signal preceding the at least one of the plurality of sensor signals and the second sensor signal following the at least one of the plurality of sensor signals.

15. The computer program product of claim 14, wherein the computer readable program instructions cause the processor to calculate an indication of the reliability of the monitored ambient air pollutant concentration over the monitoring period based on a number of sensor signals for which the determined orientation of the portable air pollutant sensor device lies outside the defined range of orientations during said monitoring period.

**FIG. 1**

**FIG. 2**

**FIG. 3**

300

**FIG. 4**

301

303

305

307

309

311

Y

N

321

323

325

N

Y

327

329

300

**FIG. 5**

**FIG. 6**

FIG. 7

EP 3 428 639 A1

Europäisches
Patentamt

European
Patent Office

Office européen
des brevets

# EUROPEAN SEARCH REPORT

Application Number

EP 17 18 1221

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MATTHIAS BUDDE ET AL: "Investigating the use of commodity dust sensors for the embedded measurement of particulate matter", NETWORKED SENSING SYSTEMS (INSS), 2012 NINTH INTERNATIONAL CONFERENCE ON, IEEE, 11 June 2012 (2012-06-11), pages 1-4, XP032206921, DOI: 10.1109/INSS.2012.6240545 ISBN: 978-1-4673-1784-9 * III. Experiments; figure 1 * | 1-15 | INV.<br>G01N33/00<br>G01N15/14<br>G01N21/00<br>G01N15/00<br>B01D46/44 |
| Y | MATTHIAS BUDDE ET AL: "The TECO Envboard: A mobile sensor platform for accurate urban sensing And more", NETWORKED SENSING SYSTEMS (INSS), 2012 NINTH INTERNATIONAL CONFERENCE ON, IEEE, 11 June 2012 (2012-06-11), pages 1-2, XP032206949, DOI: 10.1109/INSS.2012.6240573 ISBN: 978-1-4673-1784-9 * I. Introduction; table 1 * | 1-15 | |
| Y,D | JP 2016 212024 A (PANASONIC IP MAN CORP) 15 December 2016 (2016-12-15) * paragraphs [0019], [0024] * | 1-15 | TECHNICAL FIELDS SEARCHED (IPC)<br>G01N<br>B01D<br>A61B<br>F24F<br>B60H |
| A | EP 2 175 963 A2 (WOONGJIN COWAY CO LTD [KR]) 21 April 2010 (2010-04-21) * the whole document * | 1-15 | |
| A | KR 2015 0070692 A (NBREDS INC [KR]) 25 June 2015 (2015-06-25) * paragraphs [0003] - [0005]; claims; figures * | 1-15 | |

-/--

The present search report has been drawn up for all claims

| Place of search | Date of completion of the search | Examiner |
|---|---|---|
| The Hague | 7 November 2017 | Kraus, Leonie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 17 18 1221

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 106 596 363 A (BEIJING XIAOMI MOBILE SOFTWARE CO LTD; SMARTMI CO LTD) 26 April 2017 (2017-04-26) * paragraphs [0073], [0149] - [0157] * ----- | 1-15 | |
| A | MATTHIAS BUDDE ET AL: "Enabling low-cost particulate matter measurement for participatory sensing scenarios", MOBILE AND UBIQUITOUS MULTIMEDIA, ACM, 2 PENN PLAZA, SUITE 701 NEW YORK NY 10121-0701 USA, 2 December 2013 (2013-12-02), pages 1-10, XP058036385, DOI: 10.1145/2541831.2541859 ISBN: 978-1-4503-2648-3 * abstract * ----- | 1-15 | |
| A | Sanyo: "SERVICE MANUAL Air Purifier ABC-VW24", , 1 July 2007 (2007-07-01), XP055422190, Retrieved from the Internet: URL:http://s1.manualzz.com/store/data/0063 07436.pdf?key=75d820478344ec7ca68d24b7f4e2 e391&r=1&fn=6307436.pdf&t=1510045914280&p= 86400 [retrieved on 2017-11-07] * page 12 * ----- | 1-15 | |
| | | | TECHNICAL FIELDS SEARCHED (IPC) |
| A | Matthias Budde: "TECO Envboard ¦ TECO - Technology for Pervasive Computing", , 5 December 2012 (2012-12-05), XP055422549, Retrieved from the Internet: URL:http://www.teco.edu/research/teco-envb oard/ [retrieved on 2017-11-07] * the whole document * ----- -/-- | 1-15 | |

The present search report has been drawn up for all claims

| Place of search | Date of completion of the search | Examiner |
|---|---|---|
| The Hague | 7 November 2017 | Kraus, Leonie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Matthias Budde ET AL: "Demo: Handheld Particulate Matter Measurements with COTS Sensors", The 10th International Conference on Pervasive Computing (Pervasive 2012), 1 January 2012 (2012-01-01), XP055422551, Retrieved from the Internet: URL:http://www.teco.edu/~budde/publications/pervasive2012demo_budde.pdf [retrieved on 2017-11-07] * the whole document * | 1-15 | |

TECHNICAL FIELDS
SEARCHED (IPC)

The present search report has been drawn up for all claims

| Place of search | Date of completion of the search | Examiner |
|---|---|---|
| The Hague | 7 November 2017 | Kraus, Leonie |

EPO FORM 1503 03.82 (P04C01)

## ANNEX TO THE EUROPEAN SEARCH REPORT
## ON EUROPEAN PATENT APPLICATION NO.

EP 17 18 1221

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2017

| Patent document cited in search report | | Publication date | Patent family member(s) | | Publication date |
|---|---|---|---|---|---|
| JP 2016212024 | A | 15-12-2016 | NONE | | |
| EP 2175963 | A2 | 21-04-2010 | CN | 101801498 A | 11-08-2010 |
| | | | EP | 2175963 A2 | 21-04-2010 |
| | | | KR | 20090015835 A | 12-02-2009 |
| | | | US | 2011120211 A1 | 26-05-2011 |
| | | | WO | 2009020355 A2 | 12-02-2009 |
| KR 20150070692 | A | 25-06-2015 | NONE | | |
| CN 106596363 | A | 26-04-2017 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016212024 A
- JP 2008145353 A
- CN 104089858 A
- CN 203551443 U
- CN 203824872 U
- CN 105021501 A